# EUROPEAN PATENT SPECIFICATION

(11) **EP 1 401 374 B1**
(45) Date of publication and mention of the grant of the patent: **31.03.2010**
(21) Application number: 01274606.1
(22) Date of filing: 14.11.2001
(51) Int. Cl.: A61K 9/14, A61K 9/22, A61K 9/50, A61K 47/42, C07K 1/02, C07K 1/13

(54) **A NOVEL PHARMACEUTICAL COMPOUND CONTAINING ATENOLOL AND METHODS OF MAKING AND USING SAME**
NEUE PHARMAZEUTISCHE VERBINDUNG MIT ATENOLOL UND VERFAHREN ZU IHRER HERSTELLUNG UND VERWENDUNG
NOUVEAU COMPOSE PHARMACEUTIQUE CONTENANT D'ATENOLOL ET PROCEDES DE FABRICATION ET D'UTILISATION ASSOCIES

(30) Priority: 14.11.2000 US 274622 P; 14.11.2000 US 247614 P; 14.11.2000 US 247926 P; 14.11.2000 US 247610 P; 14.11.2000 US 247558 P; 14.11.2000 US 247607 P; 14.11.2000 US 247700 P; 14.11.2000 US 247930 P; 14.11.2000 US 247630 P; 14.11.2000 US 247613 P; 14.11.2000 US 247621 P; 14.11.2000 US 247611 P; 14.11.2000 US 247801 P; 14.11.2000 US 247561 P; 14.11.2000 US 247634 P; 14.11.2000 US 247633 P; 14.11.2000 US 247805 P; 14.11.2000 US 247617 P; 14.11.2000 US 247698 P; 14.11.2000 US 247800 P; 14.11.2000 US 247608 P; 14.11.2000 US 247615 P; 14.11.2000 US 247556 P; 14.11.2000 US 247807 P; 14.11.2000 US 247802 P; 14.11.2000 US 247803 P; 14.11.2000 US 247832 P; 14.11.2000 US 247797 P; 14.11.2000 US 247595 P; 14.11.2000 US 247702 P; 14.11.2000 US 247701 P; 14.11.2000 US 247560 P; 14.11.2000 US 247632 P; 14.11.2000 US 247804 P; 14.11.2000 US 247929 P; 14.11.2000 US 247927 P; 14.11.2000 US 247798 P; 14.11.2000 US 247612 P; 14.11.2000 US 247928 P; 14.11.2000 US 247833 P; 14.11.2000 US 247799 P; 14.11.2000 US 247620 P; 14.11.2000 US 247606 P; 14.11.2000 US 247635 P; 14.11.2000 US 247609 P; 14.11.2000 US 247616 P; 14.11.2000 US 247699 P; 14.11.2000 US 247631 P; 14.11.2000 US 247559 P; 14.11.2000 US 247594 P; 14.11.2000 US 247628 P; 14.11.2000 US 247719 P; 14.11.2000 US 247718 P; 14.11.2000 US 247717 P; 14.11.2000 US 247716 P; 14.11.2000 US 247754 P; 14.11.2000 US 247753 P; 14.11.2000 US 247752 P; 14.11.2000 US 247751 P; 14.11.2000 US 247750 P; 14.11.2000 US 247749 P; 14.11.2000 US 247748 P; 14.11.2000 US 247747 P; 14.11.2000 US 247796 P; 14.11.2000 US 247815 P; 14.11.2000 US 247814 P; 14.11.2000 US 247813 P; 14.11.2000 US 247812 P; 14.11.2000 US 247811 P; 14.11.2000 US 247810 P; 14.11.2000 US 247809 P; 14.11.2000 US 247808 P; 14.11.2000 US 247885 P; 14.11.2000 US 247884 P; 14.11.2000 US 247883 P; 14.11.2000 US 247882 P; 14.11.2000 US 247881 P; 14.11.2000 US 247880 P; 14.11.2000 US 247879 P; 14.11.2000 US 247878 P; 14.11.2000 US 247826 P; 14.11.2000 US 247835 P; 14.11.2000 US 247834 P; 14.11.2000 US 247897 P; 14.11.2000 US 247896 P; 14.11.2000 US 247895 P; 14.11.2000 US 247894 P; 14.11.2000 US 247901 P; 14.11.2000 US 247900 P; 14.11.2000 US 247899 P; 14.11.2000 US 247898 P; 14.11.2000 US 247903 P; 14.11.2000 US 247902 P; 14.11.2000 US 247919 P; 14.11.2000 US 247918 P; 14.11.2000 US 247917 P; 14.11.2000 US 247916 P; 14.11.2000 US 247915 P; 14.11.2000 US 247914 P; 14.11.2000 US 247913 P; 14.11.2000 US 247912 P; 14.11.2000 US 247911 P; 14.11.2000 US 247910 P; 14.11.2000 US 247877 P; 14.11.2000 US 247876 P; 14.11.2000 US 247707 P; 14.11.2000 US 247706 P; 14.11.2000 US 247705 P; 14.11.2000 US 247704 P; 14.11.2000 US 247703 P; 14.11.2000 US 247692 P; 14.11.2000 US 247691 P; 14.11.2000 US 247690 P; 14.11.2000 US 247689 P; 14.11.2000 US 247688 P; 14.11.2000 US 247687 P; 14.11.2000 US 247686 P; 14.11.2000 US 247685 P; 14.11.2000 US 247684 P; 14.11.2000 US 247683 P; 14.11.2000 US 247694 P; 14.11.2000 US 247693 P; 14.11.2000 US 247712 P; 14.11.2000 US 247711 P; 14.11.2000 US 247710 P; 14.11.2000 US 247709 P; 14.11.2000 US 247708 P; 14.11.2000 US 247697 P; 14.11.2000 US 247696 P; 14.11.2000 US 247695 P; 14.11.2000 US 247565 P; 14.11.2000 US 247564 P; 14.11.2000 US 247545 P; 14.11.2000 US 247546 P; 14.11.2000 US 247547 P; 14.11.2000 US 247548 P; 14.11.2000 US 247568 P; 14.11.2000 US 247570 P; 14.11.2000 US 247580 P; 14.11.2000 US 247555 P; 14.11.2000 US 247554 P; 14.11.2000 US 247553 P; 14.11.2000 US 247552 P; 14.11.2000 US 247551 P; 14.11.2000 US 247682 P; 14.11.2000 US 247681 P; 14.11.2000 US 247680 P; 14.11.2000 US 247679 P; 14.11.2000 US 247678 P; 14.11.2000 US 247677 P; 14.11.2000 US 247676 P; 14.11.2000 US 247655 P; 14.11.2000 US 247645 P; 14.11.2000 US 247656 P; 14.11.2000 US 247629 P; 14.11.2000 US 247627 P; 14.11.2000 US 247626 P; 14.11.2000 US 247625 P; 14.11.2000 US 247624 P; 14.11.2000 US 247806 P; 14.11.2000 US 247794 P; 14.11.2000 US 247793 P; 14.11.2000 US 247792 P; 14.11.2000 US 247791 P; 14.11.2000 US 247790 P; 14.11.2000 US 247789 P; 14.11.2000 US 247788 P; 14.11.2000 US 247787 P; 14.11.2000 US 247786 P; 14.11.2000 US 247785 P; 14.11.2000 US 247784 P; 14.11.2000 US 247783 P; 14.11.2000 US 247782 P; 14.11.2000 US 247781 P; 14.11.2000 US 247780 P; 14.11.2000 US 247779 P; 14.11.2000 US 247778 P; 14.11.2000 US 247777 P; 14.11.2000 US 247776 P; 14.11.2000 US 247775 P; 14.11.2000 US 247774 P; 14.11.2000 US 247773 P; 14.11.2000 US 247772 P; 14.11.2000 US 247771 P; 14.11.2000 US 247770 P; 14.11.2000 US 247769 P; 14.11.2000 US 247768 P; 14.11.2000 US 247767 P; 14.11.2000 US 247766 P; 14.11.2000 US 247871 P; 14.11.2000 US 247872 P; 14.11.2000 US 247873 P; 14.11.2000 US 247874 P; 14.11.2000 US 247875 P; 14.11.2000 US 247981 P; 14.11.2000 US 247982 P; 14.11.2000 US 247983 P; 14.11.2000 US 247984 P; 14.11.2000 US 247745 P; 14.11.2000 US 247744 P; 14.11.2000 US 247743 P; 14.11.2000 US 247742 P; 14.11.2000 US 247623 P; 14.11.2000 US 247985 P; 14.11.2000 US 247840 P; 14.11.2000 US 247839 P; 14.11.2000 US 247838 P; 14.11.2000 US 247837 P; 14.11.2000 US 247836 P; 14.11.2000 US 247889 P; 14.11.2000 US 247890 P; 14.11.2000 US 247891 P; 14.11.2000 US 247892 P; 14.11.2000 US 247893 P; 14.11.2000 US 247741 P; 14.11.2000 US 247740 P; 14.11.2000 US 247739 P; 14.11.2000 US 247738 P; 14.11.2000 US 247737 P; 14.11.2000 US 247736 P; 14.11.2000 US 247735 P; 14.11.2000 US 247734 P; 14.11.2000 US 247733 P; 14.11.2000 US 247732 P; 14.11.2000 US 247731 P; 14.11.2000 US 247730 P; 14.11.2000 US 247728 P; 14.11.2000 US 247729 P; 14.11.2000 US 247727 P; 14.11.2000 US 247726 P; 14.11.2000 US 247761 P; 14.11.2000 US 247760 P; 14.11.2000 US 247759 P; 14.11.2000 US 247758 P; 14.11.2000 US 247757 P; 14.11.2000 US 247756 P; 14.11.2000 US 247765 P; 14.11.2000 US 247764 P; 14.11.2000 US 247763 P; 14.11.2000 US 247762 P; 14.11.2000 US 247755 P; 14.11.2000 US 247746 P; 14.11.2000 US 247725 P; 14.11.2000 US 247724 P; 14.11.2000 US 247723 P; 14.11.2000 US 247722 P; 14.11.2000 US 247721 P; 14.11.2000 US 247720 P
(43) Date of publication of application: 31.03.2004
(73) Proprietor: Shire LLC, Florence, KY 41042 (US)
(72) Inventor: PICARIELLO, Thomas, Blacksburg, VA 24060 (US)
(74) Representative: Atkinson, Jonathan David Mark
(86) International application number: PCT/US2001/043089
(87) International publication number: WO 2003/034980

(56) References cited:
- WO-A-99/49901
- US-A- 5 534 496
- US-A- 5 846 743
- US-A- 5 898 033
- US-A- 5 948 750
- US-A- 6 030 941

## Description

### FIELD OF THE INVENTION

The present invention relates to active agent delivery systems and, more specifically, to compositions that comprise polypeptides covalently attached to active agents and methods for protecting and administering active agents.

### BACKGROUND OF THE INVENTION

The novel pharmaceutical compound of the present invention is useful in accomplishing one or more of the following goals: enhancement of the chemical stability of the original compound; alteration of the release profile of an orally administered product; enhanced digestion or absorption; targeted delivery to particular tissue/cell type; and provision for an oral dosage form when none exists. The novel pharmaceutical compound may contain one or more of the following: another active pharmaceutical agent, an adjuvant, or an inhibitor.

Active agent delivery systems are often critical for the effective delivery of a biologically active agent (active agent) to the appropriate target. The importance of these systems becomes magnified when patient compliance and active agent stability are taken under consideration. For instance, one would expect patient compliance to increase markedly if an active agent is administered orally in lieu of an injection or another invasive technique. Increasing the stability of the active agent, such as prolonging shelf life or survival in the stomach, will assure dosage reproducibility and perhaps even reduce the number of dosages required which could improve patient compliance.

Absorption of an orally administered active agent is often blocked by the harshly acidic stomach milieu, powerful digestive enzymes in the GI tract, permeability of cellular membranes and transport across lipid bilayers. Incorporating adjuvants such as resorcinol, surfactants, polyethylene glycol (PEG) or bile acids enhance permeability of cellular membranes. Microencapsulating active agents using protenoid microspheres, liposomes or polysaccharides have been effective in abating enzyme degradation of the active agent. Enzyme inhibiting adjuvants have also been used to prevent enzyme degradation. Enteric coatings have been used as a protector of pharmaceuticals in the stomach.

Active agent delivery systems also provide the ability to control the release of the active agent. For example, formulating diazepam with a copolymer of glutamic acid and aspartic acid enables a sustained release of the active agent. As another example, copolymers of lactic acid and glutaric acid are used to provide timed release of human growth hormone. A wide range of pharmaceuticals purportedly provide sustained release through microencapsulation of the active agent in amides of dicarboxylic acids, modified amino acids or thermally condensed amino acids. Slow release rendering additives can also be intermixed with a large array of active agents in tablet formulations.

Each of these technologies imparts enhanced stability and time-release properties to active agent substances. Unfortunately, these technologies suffer from several shortcomings. Incorporation of the active agent is often dependent on diffusion into the microencapsulating matrix, which may not be quantitative and may complicate dosage reproducibility. In addition, encapsulated drugs rely on diffusion out of the matrix, which is highly dependant on the water solubility of the active agent. Conversely, water-soluble microspheres swell by an infinite degree and, unfortunately, may release the active agent in bursts with little active agent available for sustained release. Furthermore, in some technologies, control of the degradation process required for active agent release is unreliable. For example, an enterically coated active agent depends on pH to release the active agent and, as such, is difficult to control the rate of release.

In the past, use has been made of amino acid side chains of polypeptides as pendant groups to which active agents can be attached. These technologies typically require the use of spacer groups between the amino acid pendant group and the active agent. The peptide-drug conjugates of this class of drug delivery system rely on enzymes in the bloodstream for the release of the drug and, as such, are not used for oral administration. Examples of timed and targeted release of injectable or subcutaneous pharmaceuticals include: linking of norethindrone, via a hydroxypropyl spacer, to the gamma carboxylate of polyglutamic acid; and linking of nitrogen mustard, via a peptide spacer, to the gamma carbamide of polyglutamine. Dexamethasone has been covalently attached directly to the beta carboxylate of polyaspartic acid without a spacer group. This prodrug formulation was designed as a colon-specific drug delivery system where the drug is released by bacterial hydrolytic enzymes residing in the large intestines. The released dexamethasone active agent, in turn, was targeted to treat large bowel disorders and was not intended to be absorbed into the bloodstream. Yet another technology combines the advantages of covalent drug attachment with liposome formation where the active ingredient is attached to highly ordered lipid films (known as HARs) via a peptide linker. Thus, there has been no drug delivery system, heretofore reported, that incorporates the concept of attaching an active ingredient to a polypeptide pendant group with its targeted delivery into the bloodstream via oral administration.

It is also important to control the molecular weight, molecular size and particle size of the active agent delivery system. Variable molecular weights have unpredictable diffusion rates and pharmacokinetics. High molecular weight carriers are digested slowly or late, as in the case of naproxen-linked dextran, which is digested almost exclusively in the colon by bacterial enzymes. High molecular weight microspheres usually have high moisture content which may present a problem with water labile active ingredients. Particle size not only becomes a problem with injectable drugs, as in the HAR application, but absorption through the brush-border membrane of the intestines is limited to less than 5 microns.

US 5 846 743 discloses polyphosphoinositide binding peptides for intracellular drug delivery obtained by covalently coupling an amino terminal blocking group to a transport-mediating peptide to form a carrier molecule.

US 6 030 941 relates to a polymer composition for delivering substances in living organisms, comprising polypeptides which exhibit solubility in both hydrophilic and lipophilic environments.

Compositions which enhance epithelial drug transport, using a peptide having a protective group, which is covalently bonded to said drug, are disclosed in US 5 534 496.

### SUMMARY OF THE INVENTION

The present invention provides covalent attachment of atelonol, hereafter referred to as the active agent, to a polymer of peptides or amino acids. The invention is distinguished from the above mentioned technologies by virtue of covalently attaching the active agent to the N-terminus, the C-terminus or directly to the amino acid side chain of an oligopeptide or polypeptide, also referred to herein as a carrier peptide. In certain applications, the polypeptide will stabilize the active agent, primarily in the stomach, through conformational protection. In these applications, delivery of the active agent is controlled, in part, by the kinetics of unfolding of the carrier peptide. Upon entry into the upper intestinal tract, indigenous enzymes release the active ingredient for absorption by the body by selectively hydrolyzing the peptide bonds of the carrier peptide. This enzymatic action introduces a second order sustained release mechanism.

Alternatively, the present invention provides a pharmaceutical composition comprising the active agent microencapsulated by a polypeptide.

The invention provides a composition comprising a polypeptide and the active agent covalently attached to the polypeptide. The polypeptide is a homopolymer of one of the twenty naturally occurring amino acids, or a heteropolymer of two or more naturally occurring amino acids.

The active agent preferably is covalently attached to a side chain, the N-terminus or the C-terminus of the polypeptide. In a preferred embodiment, the active agent is a carboxylic acid and is covalently attached to the N-terminus of the polypeptide. In another preferred embodiment, the active agent is an amine and is covalently attached to the C-terminus of the polypeptide. The active agent is an alcohol and is covalently attached to the C-terminus of the polypeptide. In yet another preferred embodiment, the active agent is an alcohol and is covalently attached to the N-terminus of the polypeptide.

The composition of the invention can also include one or more of a microencapsulating agent, an adjuvant and a pharmaceutically acceptable excipient. The microencapsulating agent can be selected from polyethylene glycol (PEG), an amino acid, a sugar and a salt. When an adjuvant is included in the composition, the adjuvant preferably activates an intestinal transporter.

Preferably, the composition of the invention is in the form of an ingestable tablet, an intravenous preparation or an oral suspension. The active agent can be conformationally protected by folding of the polypeptide about the active agent. In another embodiment, the polypeptide is capable of releasing the active agent from the composition in a pH-dependent manner.

The invention also provides a method for protecting the active agent from degradation comprising covalently attaching the active agent to a polypeptide.

The invention also provides a method for controlling release of the active agent from a composition comprising covalently attaching the active agent to the polypeptide.

The patient is a human or a non-human animal. The active agent is released from the composition by an enzyme-catalyzed release. In another preferred embodiment, the active agent is released in a time-dependent manner based on the pharmacokinetics of the enzyme-catalyzed release. In another preferred embodiment, the composition further comprises the microencapsulating agent and the active agent is released from the composition by dissolution of the microencapsulating agent, In another preferred embodiment, the active agent is released from the composition by a pH-dependmt unfolding of the polypeptide. In another preferred embodiment, the active agent is released from the composition in a sustained release. In yet another preferred embodiment, the composition further comprises an adjuvant covalently attached to the polypeptide and release of the adjuvant from the composition is controlled by the polypeptide. The adjuvant can be microencapsulated into a carrier peptide-drug conjugate for biphasic release of active ingredients.

The method for preparing a composition comprising a polypeptide and the active agent covalently attached to the polypeptide comprises the steps of:
(a) attaching the active agent to a side chain of an amino acid to form an active agent/amino acid complex;
(b) forming the active agent/amino acid complex N-carboxyanhydride (NCA) from the active agent/amino acid complex; and
(c) polymerizing the active agent/amino acid complex N-carboxyanhydride (NCA). In one variant of the method, steps (a) and (b) are repeated prior to step (c) with a second active agent. When steps (a) and (b) are repeated prior to step (c) with a second agent, the active agent and second active agent can be copolymerized in step (c). In another variant, the amino acid is glutamic acid and the active agent is released from the glutamic acid as a dimer upon a hydrolysis of the polypeptide and wherein the active agent is released from the glutamic acid by coincident intramolecular transamination. In another variant, the glutamic acid is replaced by an amino acid selected from the group consisting of aspartic acid, arginine, cysteine, lysine, threonine, and serine, and wherein the active agent is attached to the side chain of the amino acid to form an amide, a thioester, an ester, an ether, a urethane, a carbonate, an anhydride or a carbamate. In yet another variant, the glutamic acid is replaced by a synthetic amino acid with a pendant group comprising an amine, an alcohol, a sulfhydryl, an amide, a urea, or an acid functionality.

It is to be understood that both the foregoing general description and the following detailed description are exemplary, but are not restrictive, of the invention. The general applications of this invention to other active pharmaceutical agents is described in U.S. Patent Application Serial Number 09/642,820, filed August 22, 2000.

### DETAILED DESCRIPTION OF THE INVENTION

The present invention provides several benefits for active agent delivery. First, the invention can stabilize the active agent and prevent digestion in the stomach. In addition, the pharmacologic effect can be prolonged by delayed release of the active agent Furthermore, active agents can be combined to produce synergistic effects. Also, absorption of the active agent in the intestinal tract can be enhanced. The invention also allows targeted delivery of active agents to specifics sites of action.

The composition of the invention comprises a polypeptide and an active agent covalently attached to the polypeptide. The polypeptide is a homopolymer of one of the twenty naturally occurring amino acids, or a heteropolymer of two or more naturally occurring amino acids. The active agent is atenolol.

### Atenolol

Atenolol is a known pharmaceutical agent that is used in the treatment of hypertension or chronic stable angina pectoris in patients with chronic obstructive pulmonary disease (COPD) or type 1 diabetes mellitus. It is both commercially available and readily manufactured using published synthetic schemes by those of ordinary skill in the art. Its structure is:

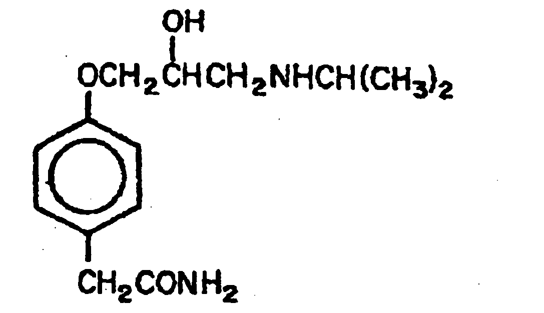

In the present invention, atenolol is covalently attached to the polypeptide via the hydroxyl group.

### Discussion

Proteins, oligopeptides and polypeptides are polymers of amino acids that have primary, secondary and tertiary structures. The secondary structure of the protein is the local conformation of the polypeptide chain and consists of helices, pleated sheets and turns. The protein's amino acid sequence and the structural constraints on the conformations of the chain determine the spatial arrangement of the molecule. The folding of the secondary structure and the spatial arrangement of the side chains constitute the tertiary structure.

Proteins fold because of the dynamics associated between neighboring atoms on the protein and solvent molecules. The thermodynamics of protein folding and unfolding are defined by the free energy of a particular condition of the protein that relies on a particular model. The process of protein folding involves, amongst other things, amino acid residues packing into a hydrophobic core. The amino acid side chains inside the protein core occupy the same volume as they do in amino acid crystals. The folded protein interior is therefore more like a crystalline solid than an oil drop and so the best model for determining forces contributing to protein stability is the solid reference state.

The major forces contributing to the thermodynamics of protein folding are Van der Waals interactions, hydrogen bonds, electrostatic interactions, configurational entropy and the hydrophobic effect. Considering protein stability, the hydrophobic effect refers to the energetic consequences of removing apolar groups from the protein interior and exposing them to water. Comparing the energy of amino acid hydrolysis with protein unfolding in the solid reference state, the hydrophobic effect is the dominant force. Hydrogen bonds are established during the protein fold process and intramolecular bonds are formed at the expense of hydrogen bonds with water. Water molecules are "pushed out" of the packed, hydrophobic protein core. All of these forces combine and contribute to the overall stability of the folded protein where the degree to which ideal packing occurs determines the degree of relative stability of the protein. The result of maximum packing is to produce a center of residues or hydrophobic core that has maximum shielding from solvent.

Since it is likely that lipophilic drugs would reside in the hydrophobic core of a peptide, it would require energy to unfold the peptide before the drug can be released. The unfolding process requires overcoming the hydrophobic effect by hydrating the amino acids or achieving the melting temperature of the protein. The heat of hydration is a destabilization of a protein. Typically, the folded state of a protein is favored by only 5-15 kcal/mole over the unfolded state. Nonetheless, protein unfolding at neutral pH and at room temperature requires chemical reagents. In fact, partial unfolding of a protein is often observed prior to the onset of irreversible chemical or conformation processes. Moreover, protein conformation generally controls the rate and extent of deleterious chemical reactions.

Conformational protection of active agents by proteins depends on the stability of the protein's folded state and the thermodynamics associated with the agent's decomposition. Conditions necessary for the agent's decomposition should be different than for protein unfolding.

Selection of the amino acids will depend on the physical properties desired. For instance, if increase in bulk or lipophilicity is desired, then the carrier polypeptide will be enriched in the amino acids in the table provided below. Polar amino acids, on the other hand, can be selected to increase the hydrophilicity of the polypeptide.

Ionizing amino acids can be selected for pH controlled peptide unfolding. Aspartic acid, glutamic acid and tyrosine carry a neutral charge in the stomach, but will ionize upon entry into the intestine. Conversely, basic amino acids, such as histidine, lysine and arginine, ionize in the stomach and are neutral in an alkaline environment.

Other factors such as π-π interactions between aromatic residues, kinking of the peptide chain by addition of proline, disulfide crosslinking and hydrogen bonding can all be used to select the optimum amino acid sequence for a given application. Ordering of the linear sequence can influence how these interactions can be maximized and is important in directing the secondary and tertiary structures of the polypeptide.

Furthermore, amino acids with reactive side chains (e.g., glutamic acid, lysine, aspartic acid, serine, threonine and cysteine) can be incorporated for attaching multiple active agents or adjuvants to the same carrier peptide. This is particularly useful if a synergistic effect between two or more active agents is desired.

As stated above, variable molecular weights of the carrier compound can have profound effects on the active agent release kinetics. As a result, low molecular weight active agent delivery systems are preferred. An advantage of this invention is that chain length and molecular weight of the polypeptide can be optimized depending on the level of conformational protection desired. This property can be optimized in concert with the kinetics of the first order release mechanism. Thus, another advantage of this invention is that prolonged release time can be imparted by increasing the molecular weight of the carrier polypeptide. Another, significant advantage of the invention is that the kinetics of active agent release is primarily controlled by the enzymatic hydrolysis of the key bond between the carrier peptide and the active agent.

Dextran is the only polysaccharide known that has been explored as a macromolecular carrier for the covalent binding of drug for colon specific drug delivery. Generally, it was only possible to load up to 1/10 of the total drug-dextran conjugate weight with drug. As stated earlier, polysaccharides are digested mainly in the colon and drug absorption is mainly limited to the colon. As compared to dextran, this invention has two major advantages. First, peptides are hydrolyzed by any one of several aminopeptidases found in the intestinal lumen or associated with the brush-border membrane and so active agent release and subsequent absorption can occur in the jejunum or the ileum. Second, the molecular weight of the carrier molecule can be controlled and, thus, active agent loading can also be controlled.

As a practical example, the following table lists the molecular weights of lipophilic amino acids (less one water molecule) and selected analgesics and vitamins.

**TABLE 1**

| **Amino acid** | **MW** | **Active agent** | **MW** |
|---|---|---|---|
| Glycine | 57 | Acetaminophen | 151 |
| Alanine | 71 | Vitamin B₆ (Pyroxidine) | 169 |
| Valine | 99 | Vitamin C (Ascorbic acid) | 176 |
| Leucine | 113 | Aspirin | 180 |
| Isoleucine | 113 | Ibuprofen | 206 |
| Phenylalanine | 147 | Retinoic acid | 300 |
| Tyrosine | 163 | Vitamin B₂ (Riboflavin) | 376 |
| | | Vitamin D₂ | 397 |
| | | Vitamin E (Tocopherol) | 431 |

Lipophilic amino acids are preferred because conformational protection through the stomach is important for the selected active agents, which were selected based on ease of covalent attachment to an oligopeptide. Eighteen was subtracted from the amino acid's molecular weight so that their condensation into a polypeptide is considered. For example, a decamer of glycine (MW=588) linked to aspirin would have a total molecular weight of 750 and aspirin would represent 24% of the total weight of the active agent delivery composition or over two times the maximum drug loading for dextran. This is only for an N- or C-terminus application, for those active agents attached to pendant groups of decaglutamic acid, for instance, a drug with a molecular weight of 180 could conceivably have a loading of 58%, although this may not be entirely practical.

The alcohol, amine or carboxylic acid group of the active agent is covalently attached to the N-terminus, the C-terminus or the side chain of the oligopeptide or polypeptide. The location of attachment depends somewhat on the functional group selection. For instance, if the active drug is a carboxylic acid (e.g., aspirin) then the N-terminus of the oligopeptide is the preferred point of attachment. If the active agent is an amine (e.g., ampicillin), then the C-terminus is the preferred point of attachment in order to achieve a stable peptide linked active agent. In both, the C- and N-terminus examples, the peptide is, in essence, extended by one monomeric unit forming a new peptide bond. If the active agent is an alcohol, then either the C-terminus or the N-terminus is the preferred point of attachment in order to achieve a stable composition. As in the example above where the alcohol, norethindrone, was covalently attached to poly(hydroxypropylglutamine), an alcohol can be converted into an alkylchloroformate with phosgene. This invention, then, pertains to the reaction of this key intermediate with the N-terminus of the peptide carrier. The active ingredient can be released from the peptide carrier by intestinal peptidases.

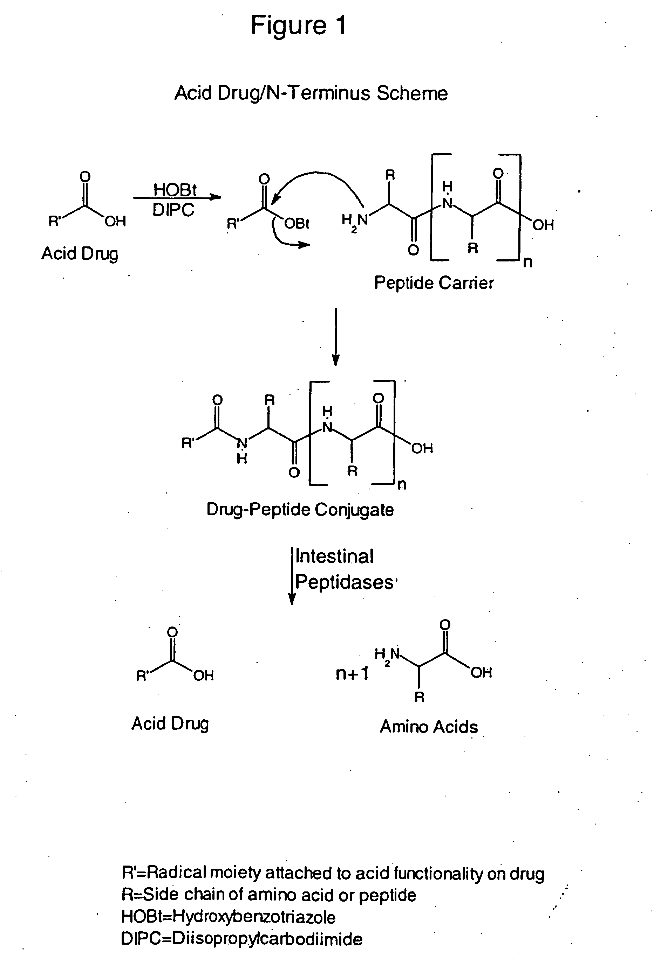

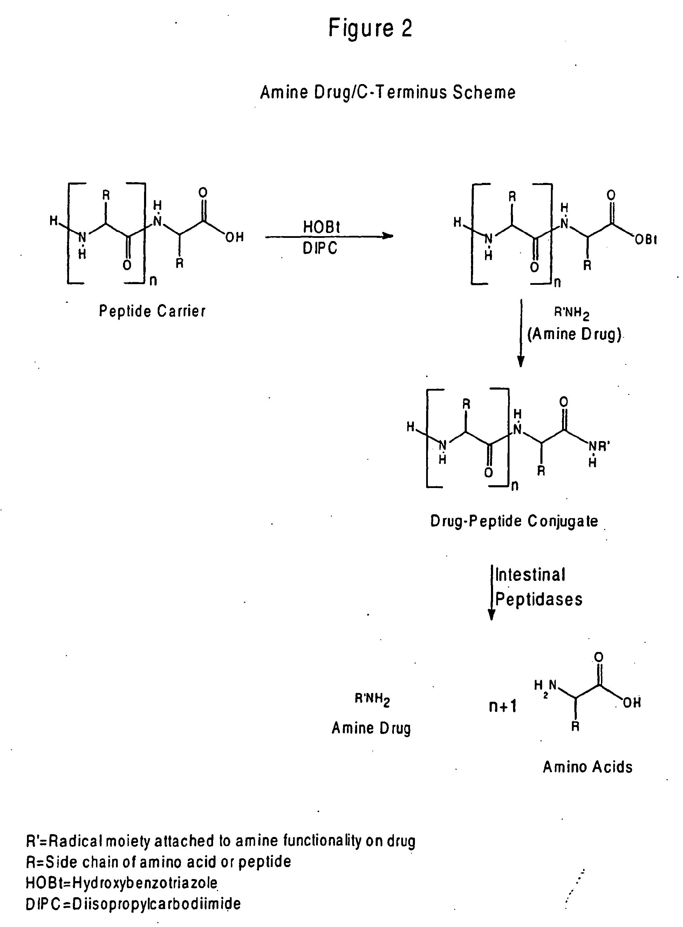

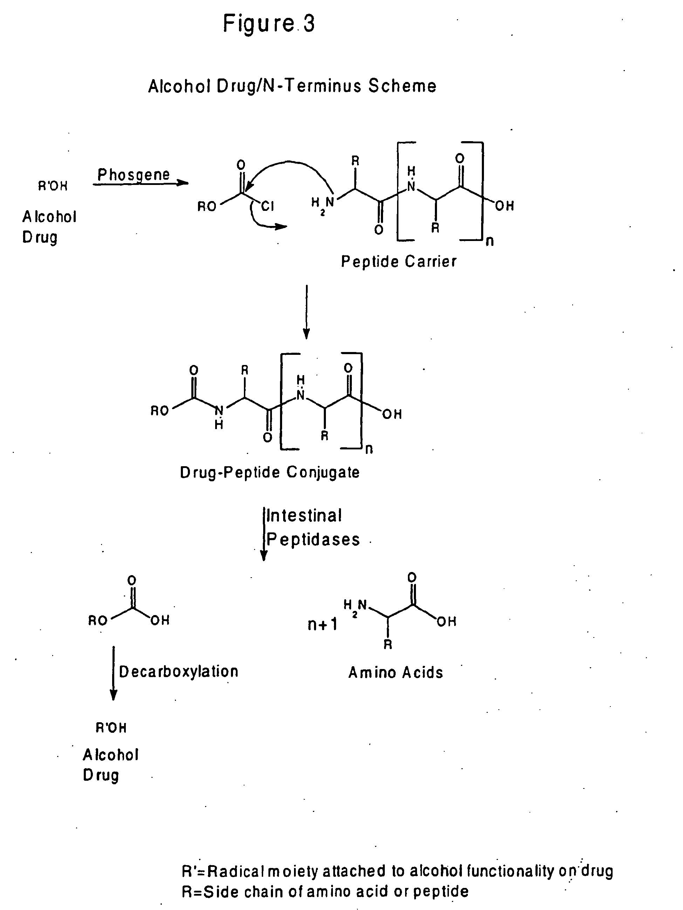

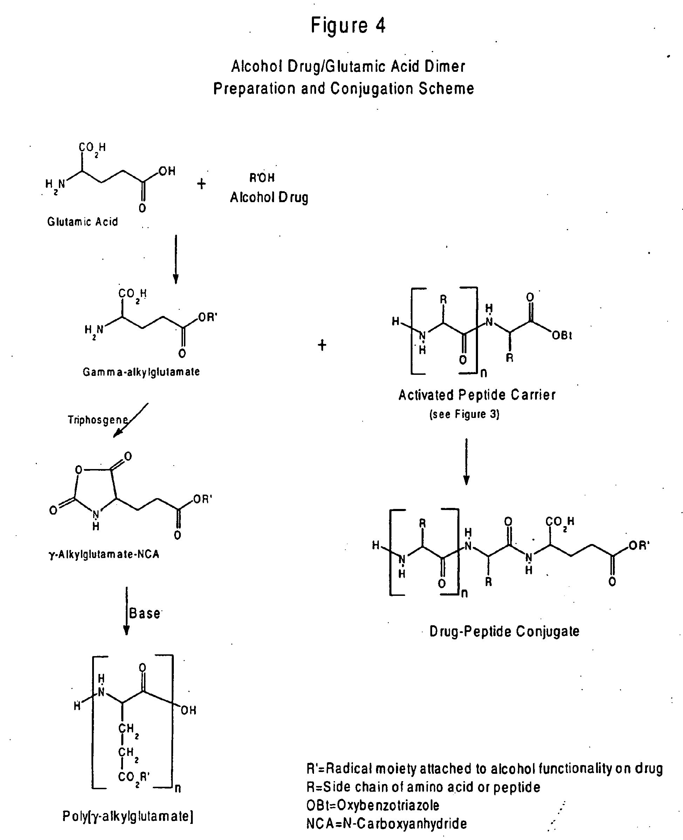

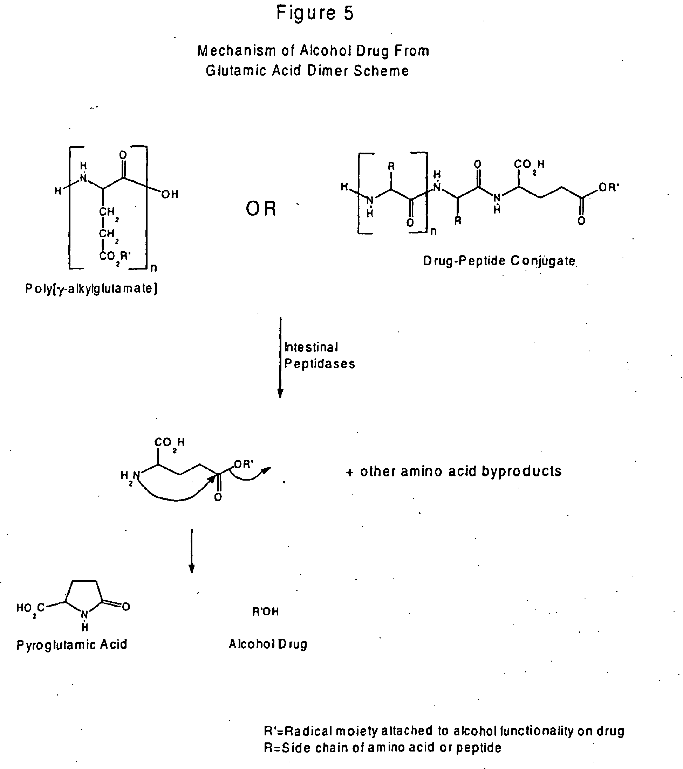

The alcohol can be selectively bound to the gamma carboxylate of glutamic acid and then this conjugate covalently attached to the C-terminus of the peptide carrier. Because the glutamic acid-drug conjugate can be considered a dimer, this product adds two monomeric units to the C-terminus of the peptide carrier where the glutamic acid moiety serves as a spacer between the peptide and the drug as shown in Fig. 4. Intestinal enzymatic hydrolysis of the key peptide bond releases the glutamic acid-drug moiety from the peptide carrier. The newly formed free amine of the glutamic acid residue will then undergo an intramolecular transamination reaction, thereby, releasing the active agent with coincident formation of pyroglutamic acid as shown in Fig. 5. Alternatively, the glutamic acid-drug dimer can be converted into the gamma ester of glutamic acid N-carboxyanhydride. This intermediate can then be polymerized, as described above, using any suitable initiator as shown in Fig. 4. The product of this polymerization is polyglutamic acid with active ingredients attached to multiple pendant groups. Hence, maximum drug loading of the carrier peptide can be achieved. In addition, other amino acid-NCA's can be copolymerized with the gamma ester glutamic acid NCA to impart specific properties to the drug delivery system.

The invention also provides a method of imparting the same mechanism of action for other polypeptides containing functional side chains. Examples include, but are not limited to, polylysine, polyasparagine, polyarginine, polyserine, polycysteine, polytyrosine, polythreonine and polyglutamine. The mechanism can translate to these polypeptides through a spacer or linker on the pendant group, which is terminated, preferably, by the glutamic acid-drug dimer. This carrier peptide-drug conjugate is distinguished from the prior art by virtue of the fact that the primary release of the drug moiety relies on peptidases and not on esterases. Alternatively, the active agent can be attached directly to the pendant group where some other indigenous enzymes in the alimentary tract can affect release.

The active agent can be covalently attached to the N-terminus, the C-terminus or the side chain of the polypeptide using known techniques. Examples of linking organic compounds to the N-terminus type of a peptide include, but are not limited to, the attachment of naphthylacetic acid to LH-RH, coumarinic acid to opioid peptides and 1,3-dialkyl-3-acyltriazenes to tetragastrin and pentagastrin. As another example, there are known techniques for forming peptide linked biotin and peptide linked acridine.

The polypeptide carrier can be prepared using conventional techniques. A preferred technique is copolymerization of mixtures of amino acid N-carboxyanhydrides. Alternatively, if a specific sequence is desired, a solid state automated peptide synthesizer can be used.

The addition of stabilizers to the composition has the potential of stabilizing the polypeptide further. Stabilizers such as sugar, amino acids, polyethylene glycol (PEG) and salts have been shown to prevent protein unfolding. In another embodiment of the invention, a pre-first order release of the active agent is imparted by microencapsulating the carrier polypeptide-active agent conjugate in a polysaccharide, amino acid complex, PEG or salts.

There is evidence that hydrophilic compounds are absorbed through the intestinal epithelia efficiently via specialized transporters. The entire membrane transport system is intrinsically asymmetric and responds asymmetrically to cofactors. Thus, one can expect that excitation of the membrane transport system will involve some sort of specialized adjuvant resulting in localized delivery of active agents. There are seven known intestinal transport systems classified according to the physical properties of the transported substrate. They include the amino acid, oligopeptide, glucose, monocarboxic acid, phosphate, bile acid and the P-glycoprotein transport systems and each has its own associated mechanism of transport. The mechanisms can depend on hydrogen ions, sodium ions, binding sites or other cofactors. The invention also allows targeting the mechanisms for intestinal epithelial transport systems to facilitate absorption of active agents.

In another embodiment of the invention, the composition includes one or more adjuvants to enhance the bioavailability of the active agent. Addition of an adjuvant is particularly preferred when using an otherwise poorly absorbed active agent. Suitable adjuvants, for example, include: papain, which is a potent enzyme for releasing the catalytic domain of aminopeptidase-N into the lumen; glycorecognizers, which activate enzymes in the BBM; and bile acids, which have been attached to peptides to enhance absorption of the peptides.

Preferably, the resultant peptide-active agent conjugate is formulated into a tablet using suitable excipients and can either be wet granulated or dry compressed.

### EXAMPLES

Compositions of the invention are, in essence, the formation of amides from acids and amines and can be prepared by the following examples.

### Acid/N-terminus conjugation

An acid bioactive agent can be dissolved in DMF under nitrogen and cooled to 0°C. The solution can then be treated with diisopropylcarbodiimide and hydroxybenzotriazole followed by the amine peptide carrier. The reaction can then be stirred for several hours at room temperature, the urea by-product filtered off, the product precipitated out in ether and purified using gel permeation chromatography (GPC) or dialysis.

### Amine/C-terminus conjugation

The peptide carrier can be dissolved in DMF under nitrogen and cooled to 0°C. The solution can then be treated with diisopropylcarbodiimide and hydroxybenzotriazole followed by the amine bioactive agent. The reaction can then be stirred for several hours at room temperature, the urea by-product filtered off, the product precipitated out in ether and purified using GPC or dialysis.

### Alcohol/N-Terminus Conjugation

In the following example the combination of the alcohol with triphosgene produces a chloroformate, which when reacted with the N-terminus of the peptide produces a carbamate. Pursuant to this, an alcohol bioactive agent can be treated with triphosgene in dry DMF under nitrogen. The suitably protected peptide carrier is then added slowly and the solution stirred at room temperature for several hours. The product is then precipitated out in ether The crude product is suitably deprotected and purified using GPC.

Other solvents, activating agents, cocatalysts and bases can be used. Examples of other solvents include dimethylsulfoxide, ethers such as tetrahydrofuran or chlorinated solvents such as chloroform. Examples of other activating agents include dicyclohexylcarbodiimide or thionyl chloride. An example of another cocatalyst is N-hydroxysuccinimide. Examples of bases include pyrrolidinopyridine, dimethylaminopyridine, triethylamine or tributylamine.

### Preparation of γ-Alkyl Glutamate

There have been over 30 different γ-alkyl glutamates prepared any one of which may be suitable for the drug alcohol of choice. For example, a suspension of glutamic acid, the alcohol and concentrated hydrochloric acid can be prepared and heated for several hours. The γ-alkyl glutamate product can be precipitated out in acetone, filtered, dried and recrystallized from hot water.

### γ-Alkyl Glutamate/C-Terminus Conjugation

The peptide carrier can be dissolved in DMF under nitrogen and cooled to 0 °C. The solution can then be treated with diisopropylcarbodiimide and hydroxybenzotriazole followed by the γ-alkyl glutamate bioactive agent. The reaction can then be stirred for several hours at room temperature, the urea by-product filtered off, the product precipitated out in ether and purified using GPC or dialysis.

### Preparation of γ-Alkyl Glutamate-NCA

γ-Alkyl glutamate can be suspended in dry THF where triphosgene is added and the mixture refluxed under a nitrogen atmosphere until the mixture becomes homogenous. The solution can be poured into heptane to precipitate the NCA product, which is filtered, dried and recrystallized from a suitable solvent.

### Preparation of Poly[γ-Alkyl Glutamate]

γ-Alkyl glutamate-NCA can be dissolved in dry DMF where a catalytic amount of a primary amine can be added to the solution until it becomes viscous (typically overnight). The product can be isolated from the solution by pouring it into water and filtering. The product can be purified using GPC or dialysis.

## Claims

1. A pharmaceutical composition comprising a polypeptide and atenolol attached through a hydroxyl group to said polypeptide, wherein said atenolol is released in the blood stream following oral administration of said pharmaceutical composition and wherein said polypeptide consists of a homopolymer of a naturally occurring aminoacid or of a heteropolymer of two or more naturally occurring aminoacids.

2. The composition according to claim 1, wherein said polypeptide is an oligopeptide.

3. The composition according to claim 1, wherein said active agent is covalently attached to a side chain or the N-terminus of said polypeptide.

4. The composition according to claim 1, wherein said active agent is covalently attached to a side chain or C-terminus.

5. The composition according to any one of claims 1 to 4 further comprising a microencapsulating agent.

6. The composition according to claim 5 wherein said mocroencapsulating agent is selected from the group consisting of polyethylene glycol (PEG), an amino acid, a sugar and a salt

7. The composition according to any one of claims 1 to 5 further comprising an adjuvant.

8. The composition according to claim 6 wherein said adjuvant activates an intestinal transporter.

9. The composition according to any one of claims 1 to 7 further comprising a pharmaceutically acceptable excipient.

10. The composition according to any one of claims 1 to 7 wherein said composition is in the form of an ingestable tablet.

11. The composition according to any one of claims 1 to 7 wherein said composition is in the form of an intravenous preparation.

12. The composition according to any one of claims 1 to 7 wherein said composition is in the form of an oral suspension.

13. The composition of claims 1 to 7 wherein said active agent is conformationally protected by folding of said polypeptide about said active agent

14. The composition of claims 1 to 7 wherein said polypeptide is capable of releasing said active agent from said composition in a pH-dependent manner.

15. A method for proctecting atenolol from degradation comprising covalently attaching atenolol through a hydroxy group to a polypeptide, wherein said atenolol is released in the blood stream following oral administration of said pharmaceuticals composition and wherein said polypeptide consists of a homopolymer of a naturally occurring aminoacid or of a heteropolymer of two or more naturally occurring aminoacids.

16. A method for controlling release of atenolol from a composition wherein said composition comprises a polypeptide, said method comprising covalently attaching atenolol through a hydroxyl group to a polypeptide, wherein said atenolol is released in the blood stream following oral administration of said pharmaceutical composition and wherein said polypeptide consists of a homopolymer of a naturally occurring aminoadd or of a heteropolymer of two or more naturally occurring aminoacids.

17. The method according to any one of claims 15 to 16 wherein said atenolol is released from said composition by an enzyme-catalysed release.

18. The method according to anyone of claim 15 to 16 wherein said atenolol is released from said composition by a pH-dependent unfolding of said polypeptide.

19. The method according to any one of claims 15 to 16 wherein said atenolol is released from said composition in a sustained release.

20. The method according to any one of claims 15 to 16 wherein said composition further comprises an adjudant covalently attached to said polypeptide and wherein release of said adjuvant from said composition is controlled by said polypeptide.

## Patentansprüche

1. Pharmazeutische Zusammensetzung, die ein Polypeptid und Atenolol aufweist, das über eine Hydroxygruppe an das Polypeptid gebunden ist, wobei das Atenolol im Anschluss an eine orale Verabreichung der pharmazeutischen Zusammensetzung in den Blutkreislauf freigesetzt wird, und wobei das Polypeptid aus einem Homopolymer aus einer natürlich vorkommenden Aminosäure oder einem Heteropolymer aus zwei oder mehr natürlich vorkommenden Aminosäuren besteht.

2. Zusammensetzung nach Anspruch 1, wobei das Polypeptid ein Oligopeptid ist.

3. Zusammensetzung nach Anspruch 1, wobei der Wirkstoff kovalent an eine Seitenkette oder den N-Terminus des Polypeptids gebunden ist.

4. Zusammensetzung nach Anspruch 1, wobei der Wirkstoff kovalent an eine Seitenkette oder den C-Terminus gebunden ist.

5. Zusammensetzung nach einem der Ansprüche 1 bis 4, die ferner ein Mikroverkapselungsmittel aufweist.

6. Zusammensetzung nach Anspruch 5, wobei das Mikroverkapselungsmittel ausgewählt ist aus der Gruppe bestehend aus Polyethylenglykol (PEG), einer Aminosäure, einem Zucker und einem Salz.

7. Zusammensetzung nach einem der Ansprüche 1 bis 5, die ferner ein Adjuvans aufweist.

8. Zusammensetzung nach Anspruch 6, wobei das Adjuvans einen intestinalen Transporter aktiviert.

9. Zusammensetzung nach einem der Ansprüche 1 bis 7, die ferner einen pharmazeutisch annehmbaren Hilfsstoff aufweist.

10. Zusammensetzung nach einem der Ansprüche 1 bis 7, wobei die Zusammensetzung in Form einer einnehmbaren Tablette vorliegt.

11. Zusammensetzung nach einem der Ansprüche 1 bis 7, wobei die Zusammensetzung in Form einer intravenösen Zubereitung vorliegt.

12. Zusammensetzung nach einem der Ansprüche 1 bis 7, wobei die Zusammensetzung in Form einer oralen Suspension vorliegt.

13. Zusammensetzung nach einem der Ansprüche 1 bis 7, wobei der Wirkstoff durch Falten des Polypeptids um den Wirkstoff konformativ geschützt ist.

14. Zusammensetzung nach einem der Ansprüche 1 bis 7, wobei das Polypeptide in der Lage ist, den Wirkstoff auf pH-abhängige Weise aus der Zusammensetzung freizusetzen.

15. Verfahren zu Schützen von Atenolol gegenüber Abbau, das das kovalente Binden von Atenolol über ein Hydroxylgruppe an ein Polypeptid aufweist, wobei das Atenolol im Anschluss an eine orale Verabreichung der pharmazeutischen Zusammensetzung in den Blutkreislauf freigesetzt wird, und wobei das Polypeptid aus einem Homopolymer aus einer natürlich vorkommenden Aminosäure oder einem Heteropolymer aus zwei oder mehr natürlich vorkommenden Aminosäuren besteht.

16. Verfahren zur Kontrolle der Freisetzung von Atenolol aus einer Zusammensetzung, wobei die Zusammensetzung ein Polypeptid aufweist, wobei das Verfahren das kovalent Binden von Atenolol über eine Hydroxygruppe an ein Polypeptid aufweist, wobei das Atenolol im Anschluss an eine orale Verabreichung der pharmazeutischen Zusammensetzung in den Blutkreislauf freigesetzt wird, und wobei das Polypeptid aus einem Homopolymer aus einer natürlich vorkommenden Aminosäure oder einem Heteropolymer aus zwei oder mehr natürlich vorkommenden Aminosäuren besteht.

17. Verfahren nach einem der Ansprüche 15 bis 16, wobei das Atenolol durch eine enzymkatalysierte Freisetzung aus der Zusammensetzung freigesetzt wird.

18. Verfahren nach einem der Ansprüche 15 bis 16, wobei das Atenolol durch ein pH-abhängiges Auffalten des Polypeptids aus der Zusammensetzung freigesetzt wird.

19. Verfahren nach einem der Ansprüche 15 bis 16, wobei das Atenolol in einer Retardfreisetzung aus der Zusammensetzung freigesetzt wird.

20. Verfahren nach einem der Ansprüche 15 bis 16, wobei die Zusammensetzung ferner ein Adjuvans aufweist, das kovalent an das Polypeptid gebunden ist, und wobei die Freisetzung des Adjuvans aus der Zusammensetzung durch das Polypeptid kontrolliert wird.

## Revendications

1. Composition pharmaceutique comprenant un polypeptide et de l'aténolol fixé par un groupe hydroxyle audit polypeptide, où ledit aténolol est libéré dans le courant sanguin après administration orale de ladite composition pharmaceutique et où le polypeptide est constitué d'un homopolymère formé d'un acide aminé naturel ou d'un hétéropolymère formé de deux acides aminés naturels ou plus.

2. Composition selon la revendication 1, où ledit polypeptide est un oligopeptide.

3. Composition selon la revendication 1, où ledit agent actif est fixé de façon covalente à une chaîne latérale ou à l'extrémité N-terminale dudit polypeptide.

4. Composition selon la revendication 1, où ledit agent actif est fixé de façon covalente à une chaîne latérale ou à une extrémité C-terminale.

5. Composition selon l'une quelconque des revendications 1 à 4, comprenant en outre un agent micro-encapsulant.

6. Composition selon la revendication 5, où ledit agent micro-encapsulant est sélectionné dans le groupe constitué du polyéthylèneglycol (PEG), d'un acide aminé, d'un sucre et d'un sel.

7. Composition selon l'une quelconque des revendications 1 à 5 comprenant en outre un adjuvant.

8. Composition selon la revendication 6, où ledit adjuvant active un agent de transport intestinal.

9. Composition selon l'une des revendications 1 à 7 comprenant en outre un excipient pharmaceutiquement acceptable.

10. Composition selon l'une quelconque des revendications 1 à 7 où ladite composition se présente sous la forme d'un comprimé ingérable.

11. Composition selon l'une quelconque des revendications 1 à 7 où ladite composition se présente sous la forme d'une préparation intraveineuse.

12. Composition selon l'une quelconque des revendications 1 à 7 où ladite composition se présente sous la forme d'une suspension orale.

13. Composition selon les revendications 1 à 7, où ledit agent actif est protégé de façon conformationnelle par pliage dudit polypeptide autour dudit agent actif.

14. Composition selon les revendications 1 à 7, où ledit polypeptide est capable de libérer ledit agent actif de ladite composition de façon dépendant du pH.

15. Procédé de protection de l'aténolol contre la dégradation comprenant la fixation covalente de l'aténolol par un groupe hydroxyle à un polypeptide, où ledit aténolol est libéré dans le courant sanguin après l'administration orale de ladite composition pharmaceutique et où ledit polypeptide est constitué d'un homopolymère formé d'un acide aminé naturel ou d'un hétéropolymère formé de deux acides aminés naturels ou plus.

16. Procédé de contrôle de la libération d'aténolol par une composition où ladite composition comprend un polypeptide, ledit procédé comprenant la fixation covalente de l'aténolol par un groupe hydroxyle à un polypeptide, où ledit aténolol est libéré dans le courant sanguin après l'administration orale de ladite composition pharmaceutique et où ledit polypeptide est constitué d'un homopolymère formé d'un acide aminé naturel ou d'un hétéropolymère formé de deux acides aminés naturels ou plus.

17. Procédé selon l'une quelconque des revendications 15 et 16 où ledit aténolol est libéré de ladite composition par libération catalysée enzymatiquement.

18. Procédé selon l'une quelconque des revendications 15 et 16 où ledit aténolol est libéré de ladite composition par dépliage dudit polypeptide dépendant du pH.

19. Procédé selon l'une quelconque des revendications 15 et 16 où ledit aténolol est libéré de ladite composition par libération prolongée.

20. Procédé selon l'une quelconque des revendications 15 et 16 où ladite composition comprend en outre un adjuvant fixé de façon covalente audit polypeptide et où la libération dudit adjuvant de ladite composition est contrôlée par ledit polypeptide.
